# EUROPEAN PATENT SPECIFICATION

(11) **EP 3 148 437 B1**
(45) Date of publication and mention of the grant of the patent: **21.03.2018**
(21) Application number: 15725330.3
(22) Date of filing: 27.05.2015
(51) Int. Cl.: A61B 5/15, A61B 5/154, A61M 5/32

(54) **BLOOD SAMPLING DEVICES AND RELATED METHODS**
BLUTENTNAHMEVORRICHTUNGEN UND ZUGEHÖRIGE VERFAHREN
DISPOSITIFS DE PRÉLÈVEMENT D'ÉCHANTILLONS DE SANG ET PROCÉDÉS ASSOCIÉS

(30) Priority: 27.05.2014 US 201462003440 P
(43) Date of publication of application: 05.04.2017
(73) Proprietor: B. Braun Melsungen AG, 34212 Melsungen (DE)
(72) Inventor: TAN, Ai-Mei, 11700 Sungai Dua Penang (MY); CHAN, Hwa Loon, 11900 Bayan Lepas Penang (MY); TEOH, Teng Sun, 11400 Air Itam Penang (MY); ZAKARIA, Mohd Zairizal, 11900 Bayan Lepas Penang (MY)
(74) Representative: Bird & Bird LLP
(86) International application number: PCT/EP2015/061704
(87) International publication number: WO 2015/181234

(56) References cited:
- WO-A1-2012/166746
- US-A- 4 887 998
- US-A1- 2004 087 875
- US-A1- 2004 210 197

## Description

### FIELD OF ART

The disclosed invention relates generally to blood sampling devices, systems and methods for taking a blood sample from a patient using a blood sample vial. Detailed discussions extend to blood sampling devices, systems and methods that include a passive needle guard mounted over a collection needle for covering the needle tip upon retraction of the needle.

### BACKGROUND

Medical care of individuals requires the widespread use of needles for taking blood samples, intravenous drug delivery, and the introduction or removal of other fluids. In the current context, the use of hypodermic needles to take blood samples has become commonplace in medicine, science, veterinary medicine, and biotechnology. The use of a hypodermic needle typically involves first inserting a needle into the patient, withdrawing a substance as required, and then removing the needle from the patient. In most applications, the withdrawn and contaminated needle must be handled very carefully during disposal to avoid a needle stick injury.

US 4,887,998 A discloses a hypodermic needle guard.

US 2004/0210197 A1 discloses a safety shielding needle assembly with passive shielding.

US 2004/0087875 A1 discloses a safety device for blood collection.

WO 2012/166746 A1 discloses an blood collection safety device.

To help prevent health care workers from becoming injured when handling used needles, safety guards have been developed to block the tip of these needles. Indeed, needle stick protection for medical professionals has become of particular importance in recent years because of the prevalence of potentially fatal infectious diseases, such as, for example, Acquired Immune Deficiency Syndrome (AIDS) and hepatitis, that can be transmitted by the exchange of bodily fluids through inadvertent wounds caused by accidental needle tip pricks from handling used needles.

Accordingly, many kinds of needle protection devices have been devised for providing post injection needle stick protection. These devices generally fall into three basic categories: those which hide the withdrawn needle within a needle shield, those which require placement of a separate needle guard that slides along the needle to cover the needle tip, and those which include a sliding shield for covering the tip of the used needle.

### SUMMARY

A passive safety needle blood sampling device is disclosed. The safety device can comprise a housing comprising a needle holder comprising a base with a flange, a distal end wall, and a body wall having an interior diameter defining an interior cavity and an exterior diameter. The body wall defines a lengthwise longitudinal axis having an open proximal end. A cap is provided having wall structure comprising an exterior surface, an interior surface defining an interior cavity, a distal end, and a proximal end connected to a distal end of the housing. At least one catch formed upon the cap, the at least one catch comprising a projection. In an example, the projection has a rectangular section extending distally of a connection point on the cap and comprising a triangular shaped section comprising a portion extending partially radially inward into the interior cavity of the cap. A needle comprising a distal end section and a proximal end section, the distal end section extending distally of the distal end of the cap and the proximal end section of the needle extending into the interior cavity of the housing and having a deformable sleeve mounted there-over forming a multi-sampling Luer adaptor. The distal end section and the proximal end section can be formed on a single shaft or from two different shafts. A safety shield comprising a first elongated section surrounding at least a portion of the distal end section of the needle and a second enlarged section comprising an interior having a shoulder, an exterior, and a flange at a proximal end thereof for covering the needle is provided. An activator is provided for activating the safety shield. The activator can comprise a body wall structure having a distal end with a distal end wall and a proximal end with an opening through which the multi-sampling Luer adaptor extends; said activator comprising two spaced apart legs with each leg comprising a hook end and extending through the distal end wall of the housing and gripping the flange on the second enlarged section of the safety shield such that the distal end wall of the activator is spaced from the distal end wall of the housing by a starting gap in a ready to use position. A helical spring is positioned in the interior of the second enlarged section of the safety shield and compressed by the shoulder of the second enlarged section and the distal end wall of the housing for moving the safety shield. The spring is held compressed by the hook ends of the two legs gripping the flange on the safety shield. A ramped section at the distal end wall of the housing in abutting contact with the two spaced apart arms; wherein the activator is movable distally when a sampling vial is inserted into the open proximal end of the housing and pushing on the activator in a distal direction, whereupon the two legs deflect by the ramped section at the distal end wall of the housing to be further spaced from one another to release the flange on the safety shield from the gripping by the two hook ends. In some examples, the distal end wall of the activator is spaced from the distal end wall of the housing by an activated gap in a protective position, which is less than the starting gap, when the two spaced apart arms no longer grip the flange on the safety shield.

A further passive safety needle blood sampling device is disclosed. The device comprises a needle comprising a distal end comprising a distal tip and a proximal end comprising a proximal tip. The proximal end has a deformable sleeve mounted there-over forming a multi-sampling Luer adaptor. A housing is provided sized and shaped to receive a blood sampling vial and comprising a base comprising a flange and having a hollow member disposed therein and fixed in relative relation therewith; said hollow member comprising an end wall comprising a plurality of openings. A protective shield is provided comprising at least two deflectable legs each comprising a hook end coaxially disposed with the hollow member and having the two hook ends engaged with two of the openings on the hollow member. A helical spring is provided compressed on one end by the protective shield and another end by the end wall of the hollow cylinder. An activator is provided comprising a base having a central opening and two spaced apart leg elements each comprising a hook end and wherein the two spaced apart leg elements extending through a respective opening on the end wall of the hollow cylinder and the two hook ends gripping the hollow cylinder such that the base of the activator is spaced from the end wall of the hollow cylinder by a starting gap in a ready to use position. Wherein when the activator is moved distally by a blood sampling vial, the two leg elements move to press against the two hook ends on the two deflectable legs of the protective shield to release the helical spring; and wherein the base of the activator is spaced from the end wall of the hollow cylinder by an activated gap, which is less than the starting gap, when the two leg elements move to press against the two hook ends on the two deflectable legs of the protective shield.

A further feature of the present disclosure is a blood collection needle assembly comprising a housing comprising a body defining an interior cavity and having a distal wall with an opening and an open proximal end for receiving a vacutainer comprising a septum; an actuator located within the interior cavity of the housing and having an arm with an arm section extending through the opening on the distal wall for gripping a second shield section of a safety shield, which has an elongated first shield section sized for surrounding a needle having a first shaft section extending distally of the distal wall of the housing and a second shaft section extending proximally of the distal wall of the housing; a biasing spring compressed between the safety shield and the housing; and wherein the actuator is movable towards the distal wall of the housing to release the second shield section from the arm section to allow the biasing spring to expand.

The blood collection needle assembly wherein the housing can comprise a housing actuator with a tapered wall extending proximally of the distal wall for interacting with the arm of the actuator.

The blood collection needle assembly wherein the actuator can comprise a cylindrical body section comprising a distal wall with an opening and an open proximal end.

The blood collection needle assembly wherein the arm is a first arm and the assembly can further comprise a second arm and wherein the first arm and the second arm extend distally from the distal wall of the actuator.

The blood collection needle assembly can further comprise a cap disposed concentrically around the needle shield and the needle.

The blood collection needle assembly wherein the cap can comprise at least one spring arm having a raised tip for abutting the second shield section in the safety shield protective position.

The blood collection needle assembly wherein part of the elongated first shield section can extend distally of the cap in a ready position.

The blood collection needle assembly can further comprise a multi-sampling Luer adaptor disposed around the second shaft section.

The blood collection needle assembly wherein the first arm and the second arm each can extend through an opening at the distal wall of the housing to grip a flange on the second shield section.

The blood collection needle assembly wherein the spring can compress between a shoulder in the second shield section and the distal wall in a ready position.

A still yet further feature of the present disclosure is a blood collection needle assembly comprising: a housing comprising a body defining an interior cavity and an open proximal end for receiving a vacutainer comprising a septum; a housing actuator comprising a body and a base disposed inside the interior cavity of the housing, said base comprising a circumference in abutting contact with an interior surface of the interior cavity, a needle post for holding a needle comprising a first shaft end and a second shaft end, and an opening spaced from the needle post; an actuator comprising a base bar and at least one arm located within the interior cavity of the housing and having the at least one arm extending through the opening on the base of the housing actuator and the base bar spaced from the base of the housing actuator in a ready position; a needle shield slidably disposed relative to the body of the housing actuator; and a biasing spring compressed between the safety shield and the housing actuator; and wherein the base bar of the actuator is movable towards the base of the housing actuator to release the needle safety shield to allow the biasing spring to expand.

The blood collection needle assembly wherein the actuator can comprise a second arm extending distally of the base bar.

The blood collection needle assembly wherein the second arm can extend through a second opening on the base of the housing actuator.

The blood collection needle assembly wherein the body of the housing actuator can be generally cylindrical and wherein the shield can be disposed, at least in part, in an interior of the housing actuator.

The blood collection needle assembly wherein the needle shield can comprise at least two leaf springs each with a hook end and wherein each hook end is engaged to an opening formed on the body of the housing actuator.

The blood collection needle assembly wherein the biasing spring has two ends and wherein a first end of the spring can be biased by an end wall of the needle shield and a second end of the spring can be biased by the base of the housing actuator.

The blood collection needle assembly wherein the arm and the second arm of the actuator can each comprise a projection that presses against the body of the housing actuator.

The blood collection needle assembly can further comprise a multi-sampling Luer adaptor positioned over the second shaft end.

The blood collection needle assembly wherein the multi-sampling Luer adaptor can project through an opening on the base bar of the actuator.

A still yet further feature of the present disclosure is a method for manufacturing and a method for using the blood collection needle assembly disclosed herein.

### BRIEF DESCRIPTION OF THE DRAWINGS

These and other features and advantages of the present devices, systems, and methods will become appreciated as the same become better understood with reference to the specification, claims and appended drawings wherein:
FIG. 1 shows an exploded perspective view of a passive safety needle blood sampling device in accordance with aspects of the present invention.
FIG. 2 shows a close up cut-away perspective view of the device of FIG. 1 in an assembled state.
FIG. 3 shows a close up cut-away perspective view of FIG. 2 with a blood sample vial inserted into the housing.
FIG. 4 shows a close up cut-away perspective view similar to that of FIG. 3 with the protective shield activated to cover the needle.
FIG. 5 shows an exploded perspective view of a passive safety needle blood sampling device in accordance with further aspects of the present invention.
FIG. 6 shows a cross-sectional side view of the embodiment of FIG. 5 in an assembled state.
FIG. 7 shows a cross-sectional side view of FIG. 6 with a blood sample vial inserted into the housing.
FIG. 8 shows a cross-sectional side view similar to that of FIG. 7, with the protective shield activated to cover the needle.

### DETAILED DESCRIPTION

The detailed description set forth below in connection with the appended drawings is intended as a description of the presently preferred embodiments of collection assemblies provided in accordance with aspects of the present devices, systems, and methods and is not intended to represent the only forms in which the present devices, systems, and methods may be constructed or utilized. The description sets forth the features and the steps for constructing and using the embodiments of the present devices, systems, and methods in connection with the illustrated embodiments. It is to be understood, however, that the same or equivalent functions and structures may be accomplished by different embodiments that are also intended to be encompassed within the spirit and scope of the present disclosure. As denoted elsewhere herein, like element numbers are intended to indicate like or similar elements or features.

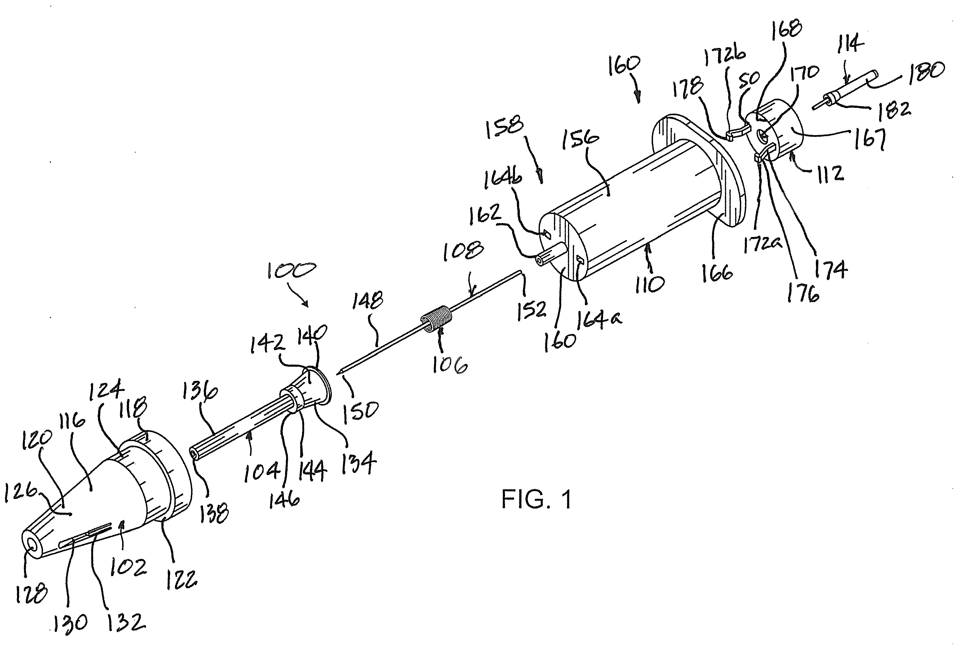

With reference now to FIG. 1, an exploded view of a collection needle assembly is shown, which is generally designated 100. In an example, the collection needle assembly 100 comprises a cap 102, a protective shield 104 slidably disposed relative to the cap 102, a helical spring 106, a needle 108, a housing or holder 110, an activator device or activator 112, and a multi-sampling Luer adaptor 114 (MSLA). The protective shield 104 is configured to automatically cover the needle 108 upon retraction of the needle from a patient or a subject without any added step and therefore may be referred to as a passive safety needle blood sampling or collection device or assembly. In other examples, a separate triggering step is provided following retraction of the needle before the shield 104 is movable over the needle to cover the needle tip making it an optional active device. When assembled, the holder 110 is sized and shaped to receive a vacuum tube and the device 100 may be used to draw a blood sample into the vacuum tube.

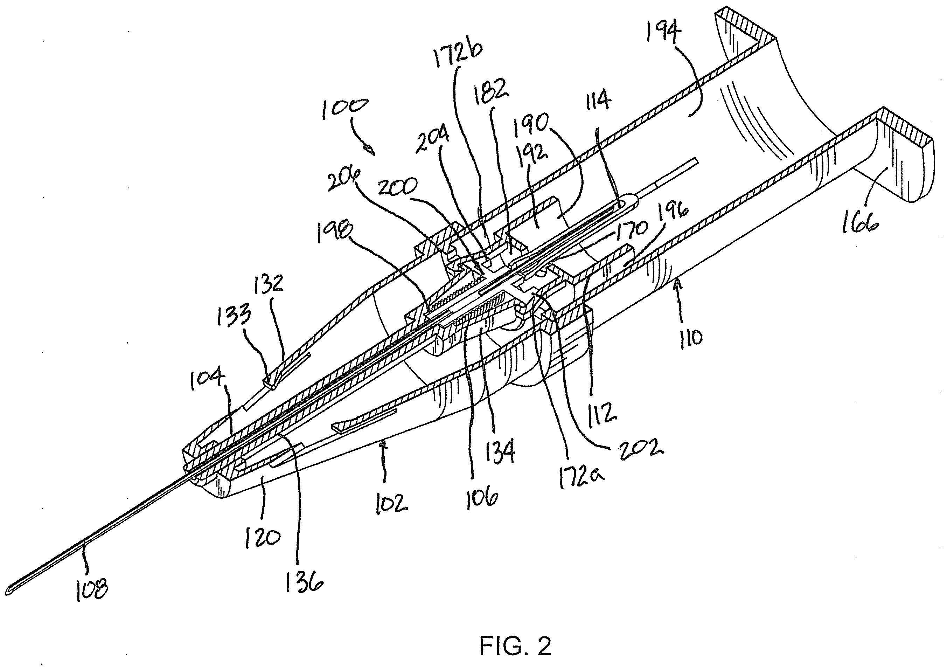

As shown, the cap 102 comprises a body 116 comprising a base 118, an elongated body chamber 120, and a shoulder 122 located therebetween. In the example shown, the base is generally round 118 and defines an opening for receiving the holder 110, as further discussed below. An internal shoulder is provided in the interior of the cap 102 and located near the external shoulder 122 for delimiting the amount of insertion of the housing into the opening of the base 118. The elongated body chamber 120 has a first generally cylindrical lower section 124 and a tapered upper section 126 comprising a distal opening 128. Two channels 130 (only one shown) are incorporated in the tapered upper section 126 with each comprising a resilient spring arm 132, which may also be referred to as a cantilevered arm 132 formed by providing a gap around three sides of an extended surface. The cantilevered arm 132, which can operate like a leaf spring, may extend proximally or distally, depending on the particular application. A raised tip 133 (FIG. 2) is provided at the end of the cantilevered arm for trapping the protective shield 104, as further discussed below. In some examples, more than two spring arms 132 are provided. In still other examples, only a single spring arm 132 is provided.

The protective shield 104 is shown with a base 134 and a shield section 136, which has a distal opening 138. The base comprises a flange 140, a tapered base body 142, which terminates in an upper base body section or slender section 144 of the base having a different contour than the tapered base body 142. A base body end surface 146 is provided at the distal end of the base 134 and has the shield section 136 extending distally thereof. The shield section 136 is generally elongated and, as shown, generally cylindrical. The shield section 136 is sized and shaped to envelope at least part of the needle 108 in the protective position to cover the needle from inadvertent needlesticks. Both the shield 104 and the cap may be made from thermoplastic, such as by plastic injection.

The needle 108 shown has a needle shaft 148, a first pointed or sharp tip 150 and a second pointed or sharp tip 152. The first sharp tip 150 is configured to penetrate a patient or subject during venipuncture while the second sharp tip 152 is configured to penetrate the septum of the vacuum tube.

The spring 106 shown is a compression spring, which is configured to compress in the ready to use position and expands to push the distal end of the cap 104 over the first sharp tip 150, as further discussed below.

The needle holder 110 has a body 156 comprising a distal end 158 and a proximal end 160. The distal end 158 comprises a closed distal end surface 160, a needle holder or post 162 for holding the needle 106, and a pair of openings or ports 164a, 164b for coupling with the activator, as further discussed below. A gripping flange 166 is provided at the proximal end of the body 156, which has an opening that leads to the interior cavity of the body 156 for receiving a vacuum tube, which is commonly referred to as a vacutainer in the relevant industry. The gripping flange provides a structure or surface for griping the assembly during venipuncture.

The activator 112 is shown with a generally cylindrical body 167 comprising a distal end wall 168 having an opening 170 and a pair of distally extending legs or arms 172a, 172b, which are spaced from one another and anchored from the distal end wall 168 at anchor points 50. The legs 172a, 172b each comprises an axially extending lower portion 174 and a tapered or flared upper section 176, which extend radially outwardly away from a central axis defined by the body 167. A lip 178 is provided at the end of each flared upper section 176. The two lips 178 are configured to hold the flange 140 on the protective shield 104, as further discussed below. The terms "legs" and "arms" are used to designate elongated structures but are not otherwise structurally limiting unless the context indicates otherwise.

The MSLA 114 is a generally elongated rubber or elastomer having a central lumen for receiving the second sharp tip 152 therein. The MSLA has an enlarged distal end 182 that serves to anchor the MSLA against the activator 112, at the opening 170 as shown in FIG. 2. In its normal expanded state, the MSLA covers the needle. However, upon pushing the septum of the vacutainer against the proximal tip 180, the MSLA collapses and the second sharp tip 152 is exposed to then puncture through the septum to form a fluid communication between the needle and the interior of the vacutainer. Upon removal of the vacutainer, the MSLA expands and the proximal tip 180 again covers the second sharp tip 152.

With reference now to FIG. 2 in addition to FIG. 1, the device 100 is shown in its pre-activation configuration with the needle 108 exposed for venipuncture, such as to draw a blod sample. As shown, the activator 112 and multi-sampling Luer adaptor 114 are placed inside the housing 110. The proximal end of the needle 108, which includes the second sharp tip 152, is covered by the multi-sampling Luer adaptor 114, which protrudes through the needle aperture or opening 170 at the distal wall 168 of the activator 112. The aperture 170 surrounds the multi-sampling Luer adaptor 114 and its opening dimension is smaller than the largest outside diameter of the enlarged distal end of the MSLA to prevent the MSLA from sliding out of the opening 170 in the proximal direction. The distal wall 168 of the activator 112 and the cylindrical wall of the body 167 are sized and shaped to receive a blood sample vial (not shown) when the needle 106 projects through the septum of the vial. In one embodiment the, hollow portion of the body 167 of the activator 112 is a hollow cylinder. In other embodiments, the cross-sectional shape of the body 167 is designed with a different shape, such oval, or polygonal, such as square, triangular, pentagon, hexagon and octagon.

In an example, the two spaced apart legs or arms 172a, 172b of the activator 112 extend through the two corresponding ports 164a, 164b at the distal wall or closed distal end surface 160 of the housing 110. The spaced apart legs are each comprised of three sections 174, 176, 178, as previously discussed. The first section 174 of each leg is generally parallel to the longitudinal axis of the device and extends from the distal wall 168 of the activator 112. The second section 176 of each leg extends distally from the first section and angles partially radially outward away from the longitudinal axis of the device. The third section 178 of each leg extends from the distal end of the second section and comprises hooks or lips that extend radially inward to the longitudinal axis of the device. The spaced apart legs 172a, 172b are configured so that they can deflect about each respective anchored point further apart from one another. In other examples, the three sections of each leg can embody different configurations, such as being tapered from the anchored point 50 and then extend generally parallel with the longitudinal axis. The two hooks or lips 178 grip against the distal wall 160 of the housing 110 to retain the activator 112 to the housing.

As shown, the body 167 of the activator 112 comprises an interior surface 190 defining a hollow interior 192 for receiving an end of a vacutainer (not shown). The wall surface of the body 167 is spaced from the wall surface 194 of the housing by a gap 196. Thus, when a vacutainer is inserted into the device 100, at least at the distal end of the vacutainer, it is held within the body section of the activator 112, which is spaced from the wall surface 194 of the housing 110 by the gap 196.

The protective shield 104 comprises a slender first section or shield section 136 which surrounds the needle 108 and in one embodiment protrudes out the distal opening 128 of the cap 102. In other embodiments, the distal end of the first section 136 may be flushed with or recessed inside the cap 102. A hollow second section or base 134 is attached to the proximal end of the slender first section 136. The hollow second section 134 comprises a flange 140 extending radially from the proximal end and is held against the closed distal end surface 160 of the housing by the lips 178 on the two legs 172a, 172b of the activator. The hollow second section or base 134 is sized and shaped to accommodate the needle holder 162 and the spring 106, which is held in a compressed state by the two hooks on the activator 112. As shown, the spring 106 is positioned over or to the outside of the needle holder 162 and held compressed at its two ends by the distal end surface 160 of the housing 110 and the shoulder 198 inside the protective shield 104. In one example, the spring is a helical spring of sufficient spring force and length to expand the protective shield 104 over the needle tip, as further discussed below.

With further reference to FIG. 2, the needle 106 is shown comprising two separate needle sections. The two needle sections are attached to the needle holder 162 from each section's blunt end and a space is provided therebetween. The needle sections may be attached using conventional means and techniques. In other embodiments, the needle 106 is a single needle with a single shaft 148 comprising two pointed ends 150, 152.

A housing actuator 200 is provided on the proximal side or interior surface of the distal end wall 160 of the housing 110. As shown, the housing actuator 200 is contoured with a ramped surface 202. The ramped surface is generally shaped as a cuboid protrusion from the proximal side of the distal end wall 160 of the housing 110. The ramped surface 202 slants radially inward from the distal end to the proximal end. The second section 176 of each of the two legs 172a, 172b rests against the ramped surface 202. In one example, the ramped surface 202 forms a continuous structure about the central region of the housing 110. In another example, the ramped surface 202 is non-continuous and is provided in the same general locations as the two axially extending legs 172a, 172b. Preferably, the ramped surface 202 and the tapered upper sections 176 of the two legs form a close surface contact with no space or gap in between. In other examples, a gap is provided. The ramped surface 202 is sized and shaped to contact the two legs without spreading or bending the legs outwardly due to the size difference.

With further reference to the activator 112 and the housing activator 200, a gap is provided between the distal end surface of the end wall 168 of the activator 112 and the proximal most edge 204 of the housing activator 200. This gap allows the activator 112 to move distally forward towards the housing activator 200 and the distal end wall 160 of the housing without being obstructed or limited by the ramped surface structure. As further discussed below, when the actuator 112 is caused to move distally forward into the gap 196, the two ramped surfaces interact to spread the two axially extending legs 172a, 172b, which will then release the flange 140 on the protective shield 104 to then permit the spring to expand.

The base 118 on the cap 102 has a bore at its proximal end with an interior diameter that is greater than the exterior diameter of the housing 110 to receive the housing. A shoulder 206 is provided in the bore of the cap to limit the extent of insertion of the housing 110 into the bore.

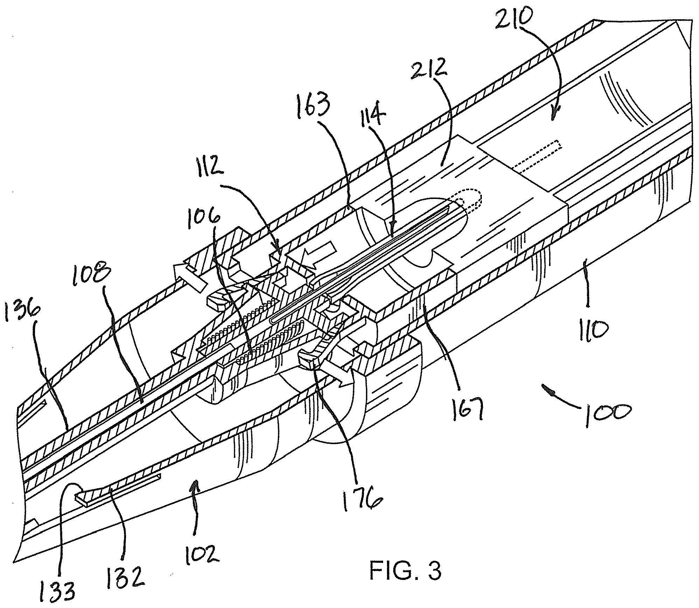

With reference now to FIG. 3, the device is shown with a blood sample vial or vacutainer 210 inserted through the open proximal end of the housing 110 (FIG. 2). The blood sample vial 210 is pushed distally until the vial 210 abuts the proximally facing edge 163 of the body 167 of the activator 112. In one example, the needle 108 is first placed into a patient's vein before the vacutainer is inserted into the open end of the housing 110. At the same time, the septum 212 located on the vacutainer pushes against the MSLA 114 to open the second pointed tip 152 of the needle, which then penetrates the septum. As the blood sample vial continues to push the activator 112 distally, the spaced apart legs 150 of the activator move correspondingly distally, sliding in contact with the ramped surface 202 on the housing actuator 200, which moves towards the distal wall 168 of the actuator 112. The ramped surface 202 eventually pushes against the first leg section 174 of the two legs 172a, 172b, and due to relative dimensions, causes the first section 174 of the spaced apart legs 172a, 172b to be driven radially outward until the tips of the hooks 178 are sufficiently spaced apart that the distance between them is greater than the exterior diameter of the flange 140 on the base 134 of the protective shield 104. At that point, the restraining force on the spring is released and the spring 106 pushes the protective shield 104 distally along the needle 108. If the needle 108 has penetrated the patient or subject, then the protective shield 104 will advance distally until the distal end of the protective shield comes into contact with the patient or the subject's skin (not shown). If the needle is outside of the patient or subject, then the spring will advance the protective shield to its most distal locked position. In the example shown, the protective shield's most distal locked position is marked by the flange moving distally of the raised tips 133 on the spring arms 132 and held there in the protective position.

Thus, aspects of the present embodiment is understood to include a blood sampling needle assembly in which a needle shield is provided and wherein the needle shield is released from its original position prior to retracting the needle from a patient's vein. The assembly is further understood to comprise an actuator configured for releasing the needle shield. As disclosed, the actuator is slidable or movable along an lengthwise axis of the assembly and has actuator features that move radially of the lengthwise axis. For example, the actuator comprises arms that move radially to release the shield from the arms' hold. In specific examples, the arms are moved by pushing them against ramped surfaces to move radially outwardly.

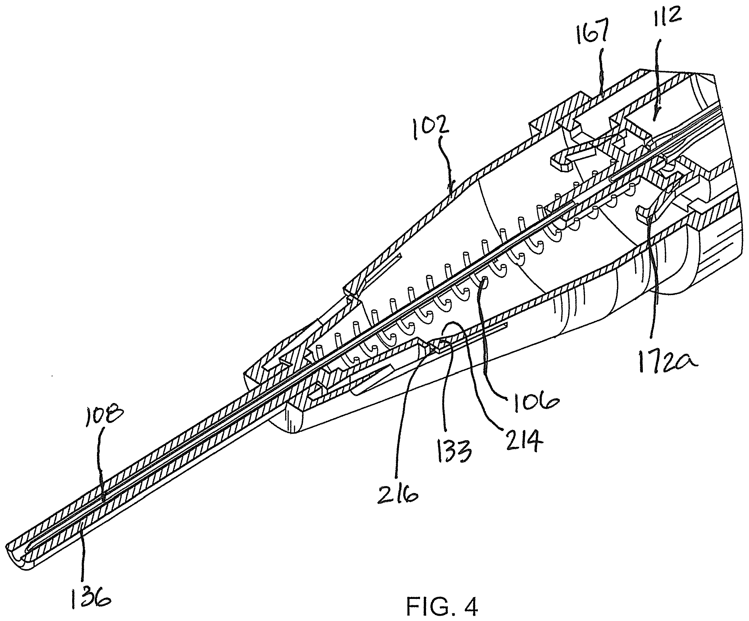

As shown in FIG. 4, when the needle 108 is removed from the patient, such as following successful sampling of one or more vacutainers, the spring 106 will continue to bias the protective shield 104 distally until the flange 140 on the proximal end of the base 134 of the protective shield 104 comes into contact with the two catches or raised tips 133 on the cap 102. Each catch 133 comprises a ramped surface 214 and a blocking end 216. The ramped surface 214 may comprises a linear slope or a complex curve. As the protective shield moves distally by the spring, the flange 140 pushes the catches 133 radially outward due to the relative dimensions of the flange outer diameter and the distance between the two catches. After the flange 140 moves past the two catches 133, they recoil and the blocking ends 216 are moved proximally of the flange to block the flange from returning to the proximal position to re-expose the needle. In one embodiment, the spring arms 132 are integral to the cap 102. In other embodiments, the spring arms 132 may be attached by various means, including the use of welding or through mechanical connections. In the shielded or protective position of FIG. 4, the protective shield 104 covers the distal tip 130 of the needle and prevents any accidental needle stick therewith.

With reference again to FIG. 3, in some examples, the body 167 of the activator is sized and shaped to ensure adequate grip on the vacutainer 210 and the needle 108 is sized and shaped to fully penetrate the septum 212 when withdrawing blood from the patient or subject. As shown, the vacutainer 210 is held inside the holder or housing 110 by the needle penetrating the septum only. The body 167 on the activator does not surround or envelope the septum 212. In other examples, the body 167 and the septum form a close fit or a slight interference fit.

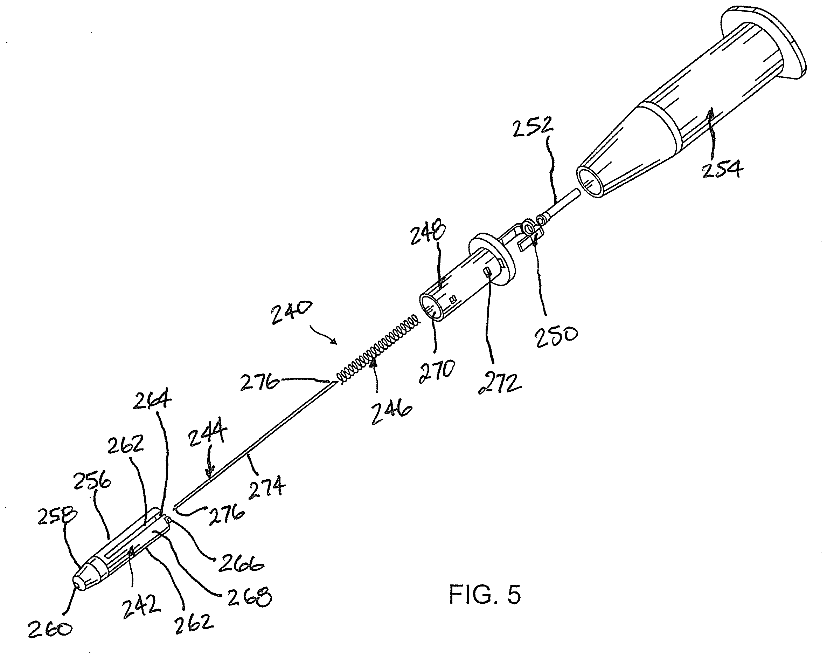

FIGs. 5-8 show another collection needle assembly 240 provided in accordance with further aspects of the present devices, systems, and assemblies. Turning initially to FIG. 5, the needle assembly 240 comprises a protective shield 242, a cannula or needle 244, a helical spring 246, a housing actuator 248, an activator device or activator 250, a multi-sampling Luer adapter (MSLA) 252 comprising a deformable sleeve, and a housing or holder 254. In the present embodiment, the housing actuator 248 and the housing 254 are two independently movable components.

In the present embodiment, the protective shield 242 has an elongated body 256 comprising a tapered nose end 258 with a distal opening 260, two or more elongated slots 262 with each terminating at the proximal end opening 264 and having a radially extending hook end 266. The slots 262 on the body 256 define flexible leaf springs 268 each with at least one hook end 266. As further discussed below, the protective shield 242 is configured to enter the distal opening 270 on the housing 248 and the leaf springs 242 are sized and shaped to deflect and then uncoil so that the hook ends 266 engage corresponding detents 272 formed with the housing 248.

The needle 244 has a needle shaft 274 and is provided with two sharpened needle tips 276 at opposite ends of the shaft 274. As further discussed below, the needle 244 is held by a needle post located inside the housing activator 248. The needle may be secured thereto via conventional means.

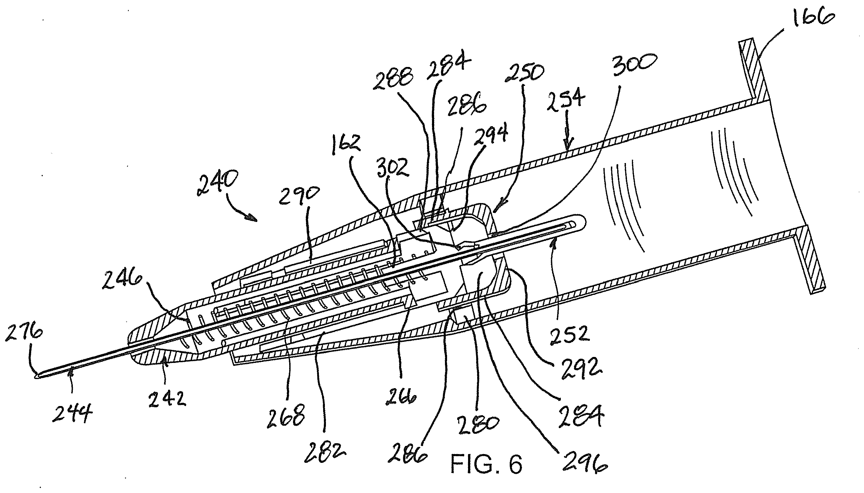

FIG. 6 shows the device 240 assembled and in its pre-activation configuration. A sharp distal tip 276 of the needle 244 is exposed through the opening 260 of the protective shield 242. The needle 244 is retained to the housing activator 248 by passing through the needle post 162, which projects axially from the base 280. The needle 244 is exposed by pulling the shield 242 in a proximal direction and compressing the helical spring 246. The spring 246 is held compressed by engaging the hook ends 266, which are located on the proximal end of the shield 242, with the female detents 272 on the housing activator 248 to compress the two spring ends of the spring 246 at one end by the end surface of the shield 242 and at the other end by the base 280 of the housing activator. As shown, the body 256 of the shield is placed inside the body 282 of the housing activator 248. The second end of the needle tip 276 is provided inside the sleeve of the MSLA 252. The deformable MSLA covers the proximal needle tip 276 and can be punctured by the proximal needle tip 276 when a user inserts a blood sample vial (not shown) into the device 240.

Also shown in FIG. 6 are the two arms 284 of the activator 250 extending through two slots 286 formed through the base 280. A projection 288 at an end of each arm 284 causes the arms to slightly deflect outwardly and biases against the exterior wall surfaces 290 of the body 282 of the activator. This prevents the arms 284 from falling back out through the two slots 286 in the proximal direction. The activator 250 further comprises a base bar 292 that is spaced from the proximal end surface 294 of the base 280 by a clearance gap 296 in the needle exposed position or ready to use position shown in FIG. 6. As further discussed below, the base bar 292 can move distally forward upon insertion of a vacutainer to reduce the clearance gap 296 to activate the needle shield. A centrally located opening 300 on the base bar 292 is provided to accommodate the MSLA 252 and the second end of the needle. The enlarged end 302 on the MSLA 252 prevents it from being displaced out through the opening 300.

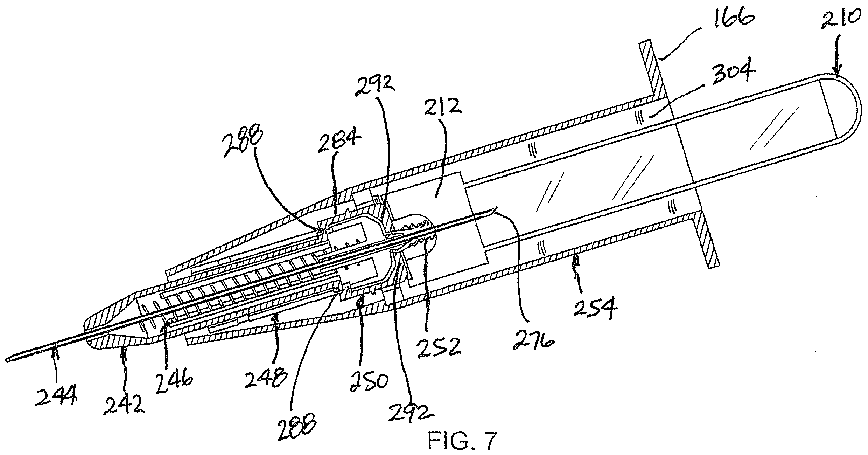

FIG. 7 shows a vacutainer 210 inserted through the proximal opening 304 of the holder 254. The vacutainer 210 is inserted until the septum 212 pushes against the MSLA 252 and continues until the MSLA 252 is compressed and the needle tip 276 punctures through the septum 212. The vacutainer 210 is further inserted such that it pushes against the base bar 292 of the activator 250 until the base bar contacts the exterior proximal end surface 294 of the base 280 or until the activator 250 is physically delimited by some other structural feature on the housing activator 248. At this point, the two projections 288 on the two arms 284 of the activator 250 are advanced over the two female detents 272 and each projection projects into a corresponding female detent to push against the hook ends 266. This causes the two projections or hook ends 266 on the shield 242 to separate from the female detents 272. However, at this point, the shield 242 does not necessarily get propelled by the spring 246. In practice, the needle 244 would first be inserted into a patient's vein and the distal end 306 of the shield 242 pushed against the skin. This prevents the shield 242 from being pushed by the spring. The spring expands upon retraction of the needle away from the patient, which is similar to the first embodiment shown with reference to FIGs. 1-4.

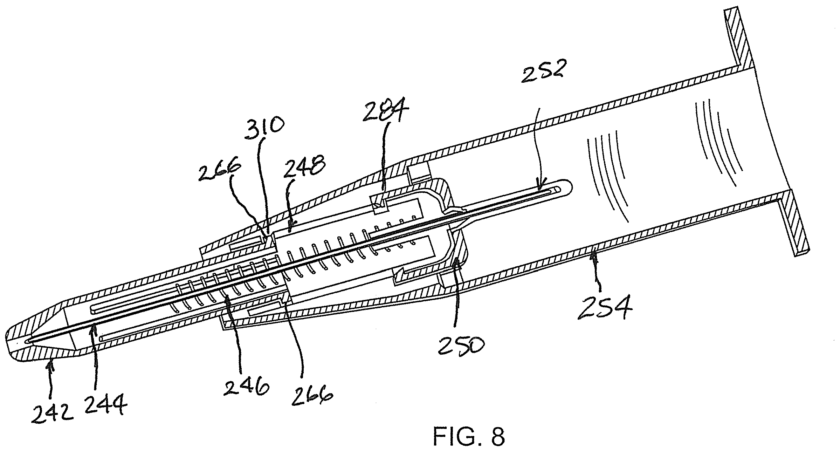

With reference now to FIG. 8, the spring 246 is allowed to expand after removing the needle 244 from the patient as the skin no longer limits movement of the distal end 306 of the shield 242. The expanding spring 246 moves the shield 242 distally until the hook ends 266 on the leaf springs 268 engage a second set of female detents 310. Once the hook ends 266 engage the distal female detents 310, which in the present embodiments are openings formed on the housing activator, the protective shield 242 covers the distal tip 276 of the needle 244 and is prevented from being compressed to re-expose the needle. Further, because there is no force to counteract the radially outward direct force of the hook ends 266, the engagement of the hook ends 266 and the distal openings 310 locks the protective shield 242 in place, covering the distal needle tip 244 and preventing needle stick injuries.

Thus, aspects of the present embodiment is understood to include a blood sampling needle assembly in which a needle shield is provided and wherein the needle shield is released from its original position prior to retracting the needle from a patient's vein. The assembly is further understood to comprise an actuator configured for releasing the needle shield. As disclosed, the actuator is slidable or movable along a lengthwise axis of the assembly and has actuator features that move radially of the lengthwise axis. For example, the actuator comprises arms that move radially to release the shield from the arms' hold. In an example, the arms are moved by biasing them in an outwardly position relative to the lengthwise axis and then allowing them to move radially inwardly relative to the lengthwise axis to release the latch on the needle shield. In another example, the arms are moved by providing openings on the needle shield so that projections on the arms are provided with room to move radially inwardly into the openings. Said movement can be configured to trigger the detents on the needle shield to release to subsequently cover the needle tip.

Although limited embodiments of the passive safety needle blood sampling or collection assemblies and their components have been specifically described and illustrated herein, many modifications and variations will be apparent to those skilled in the art. For example, the various protective shields may incorporate translucent or semi-transparent materials allowing a user to view the needle after the needle shield is released, etc. Furthermore, it is understood and contemplated that features specifically discussed for one passive safety needle blood sampling device embodiment may be adopted for inclusion with another passive safety needle blood sampling device embodiment, provided the functions are compatible. For example, a hollow seat for receiving a vial in an activator may be used in another embodiment shown with just the ring and leg elements. Another example includes elements that allow a user to detect flashback. Accordingly, it is to be understood that the safety needle blood sampling device assemblies and their components constructed according to principles of the disclosed device, system, and method may be embodied other than as specifically described herein. The invention is also defined in the following claims.

## Claims

1. A blood collection needle assembly (100, 240) comprising:
a housing (110, 248) comprising a body (156, 282) defining an interior cavity and having a distal wall (160, 280) with an opening (164a, 286) and an open proximal end for receiving a vacutainer comprising a septum;
an actuator (112, 250) located within the interior cavity of the housing (110) and having an arm (172a, 284) with an arm section (176) extending through the opening (164a, 286) on the distal wall (160, 280) for gripping a second shield section (134) of a safety shield (104, 242), which has an elongated first shield section (136) sized for surrounding a needle (108, 244) having a first shaft section extending distally of the distal wall (160, 280) of the housing (110, 248) and a second shaft section extending proximally of the distal wall (160, 280) of the housing (110, 248);
a biasing spring (106, 246) compressed between the safety shield (104, 242) and the housing (110, 248); and
wherein the actuator (112, 250) is movable towards the distal wall (160, 280) of the housing (110, 248) to release the second shield section (134) from the arm section (176) to allow the biasing spring (106, 246) to expand.

2. The blood collection needle assembly of claim 1, wherein the housing (110) has a housing actuator (200) comprising a tapered wall (202) extending proximally of the distal wall (160) for interacting with the arm (172a) of the actuator (112).

3. The blood collection needle assembly of claims 1 or 2, wherein the actuator (112, 250) comprises a cylindrical body section (167) comprising a distal wall (168) with an opening (170, 300) and an open proximal end (196).

4. The blood collection needle assembly of claim 3, wherein the arm (172a, 284) is a first arm (172a, 284) and further comprising a second arm (172b, 284) and wherein the first arm (172a, 284) and the second arm (172b, 284) extend distally from the distal wall (168) of the actuator (112, 250).

5. The blood collection needle assembly of any one of claims 1 to 4, further comprising a cap (102), disposed concentrically around the needle shield (104) and the needle (108).

6. The blood collection needle assembly of claim 5, wherein the cap (102) comprises at least one spring arm (132) having a raised tip (133) for abutting the second shield section (134) in the safety shield protective position.

7. The blood collection needle assembly of any one of claims 5 or 6, wherein part of the elongated first shield section (136) extends distally of the cap (102) in a ready position.

8. The blood collection needle assembly of any one of claims 1 to 7, further comprising a multi-sampling Luer adaptor (114) disposed around the second shaft section.

9. The blood collection needle assembly of claim 4, wherein the first arm (172a) and the second arm (172b) each extends through an opening at the distal wall (160) of the housing (110) to grip a flange (140) on the second shield section (134).

10. The blood collection needle assembly of any one of claims 1 to 9, wherein the spring (106) is compressed between a shoulder (198) in the second shield section (134) and the distal wall (160) in a ready position.

11. A blood collection needle assembly (100, 240) comprising:
a housing (102, 254) comprising a body (156) defining an interior cavity and an open proximal end for receiving a vacutainer comprising a septum;
a housing actuator (110, 248) comprising a body (156, 282) and a base (160, 280) disposed inside the interior cavity of the housing (102, 254), said base (160, 280) comprising a circumference in abutting contact with an interior surface of the interior cavity, a needle post (162) for holding a needle (108, 244) comprising a first shaft end and a second shaft end, and an opening (164a, 286) spaced from the needle post (162);
an actuator (112, 250) comprising a base bar (167, 292) and at least one arm (172a, 284) located within the interior cavity of the housing (102, 254) and having the at least one arm (172a, 284) extending through the opening (164a, 286) on the base (160, 280) of the housing actuator (110, 248) and the base bar (167, 292) spaced from the base (160, 280) of the housing actuator (110, 248) in a ready position;
a needle shield (104, 242) slidably disposed relative to the body (156, 282) of the housing actuator (110, 248); and
a biasing spring (106, 246) compressed between the needle shield (104, 242) and the housing actuator (110, 248); and
wherein the base bar (167, 292) of the actuator (112, 250) is movable towards the base (160, 280) of the housing actuator (110, 248) to release the needle shield (104, 242) to allow the biasing spring (106, 246) to expand.

12. The blood collection needle assembly of claim 11, wherein the actuator (112, 250) comprises a second arm (172b, 284) extending distally of the base bar (167, 292).

13. The blood collection needle assembly of claim 12, wherein the second arm (172b, 284) extends through a second opening (164b, 286) on the base (160, 280) of the housing actuator (110, 248).

14. The blood collection needle assembly of any one of claims 11 to 13, wherein the body (156, 282) of the housing actuator (110, 248) is generally cylindrical and wherein the needle shield (104, 242) is disposed, at least in part, in an interior of the housing actuator (110, 248).

15. The blood collection needle assembly of any one of claims 11 to 14, wherein the needle shield (242) comprises at least two leaf springs (268) each with a hook end (266) and wherein each hook end (266) is engaged to an opening (272) formed on the body (282) of the housing actuator (248).

16. The blood collection needle assembly of any one of claims 11 to 15, wherein the biasing spring (106, 246) has two ends and wherein a first end of the spring (106, 246) is biased by an end wall of the needle shield (104, 242) and a second end of the spring (246) is biased by the base (160, 280) of the housing actuator (110, 248).

17. The blood collection needle assembly of any one of claims 12 to 16, wherein the arm (172a, 284) and the second arm (172b, 284) of the actuator (112, 250) each comprises a projection (178, 288) that presses against the body (156, 282) of the housing actuator (110, 248).

18. The blood collection needle assembly of any one of claims 11 to 17, further comprising a multi-sampling Luer adaptor (114, 252) positioned over the second shaft end.

19. The blood collection needle assembly of claim 18, wherein the multi-sampling Luer adaptor (114, 252) projects through an opening (170, 300) on the base bar (167, 292) of the actuator (112, 250).

20. A method for making a blood collection needle assembly (100, 240), the method comprising:
providing a housing (102, 254) comprising a body (156) defining an interior cavity and an open proximal end, and a housing actuator (110, 248) comprising a body (156, 282) and a base (160, 280) disposed inside the interior cavity of the housing (102, 254);
abutting a circumference of said base (160, 280) with an interior surface of the interior cavity, the base (160, 280) comprising a needle post (162) for holding a needle (108, 244) comprising a first shaft end and a second shaft end, and an opening (164a, 286) spaced from the needle post (162);
locating an actuator (112, 250) comprising at least one arm (172a, 284) within the interior cavity of the housing (102, 254);
extending the at least one arm (172a, 284) through the opening (164a, 286) on the base (160, 280) of the housing actuator (110, 248);
slidably disposing a needle shield (104, 242) relative to the body (156, 282) of the housing actuator (110, 248); and
compressing a biasing spring (106, 246) between the needle shield (104, 242) and the housing actuator (110, 248); and
wherein the actuator (112, 250) is movable towards the base (160, 280) of the housing actuator (110, 248) to release the needle shield (104, 242) to allow the biasing spring (106, 246) to expand.

21. The method of claim 20, wherein the needle shield (242) comprises at least two leaf springs (268) each with a hook end (266) and wherein each hook end (266) is engaged to an opening (272) formed on the body (282) of the housing actuator (248).

22. The method of any one of claim 20 to 21, wherein the at least one arm (172a) grips a second shield section (134) of the needle shield (104), which has an elongated first shield section (136) sized for surrounding a needle (108) having a first shaft section extending distally of the distal wall (160) of the housing (110) and a second shaft section extending proximally of the distal wall (160) of the housing (110).

## Patentansprüche

1. Blutentnahmenadelanordnung (100, 240), aufweisend:
ein Gehäuse (110, 248), aufweisend einen Körper (156, 282), der einen inneren Hohlraum definiert und eine distale Wand (160, 280) mit einer Öffnung (164a, 286) und einem offenen proximalen Ende zur Aufnahme eines Vacutainers, der ein Septum aufweist, hat;
einen Aktuator (112, 250), der innerhalb des inneren Hohlraumes des Gehäuses (110) angeordnet ist und einen Arm (172a, 284) mit einem Armabschnitt (176) aufweist, der sich durch die Öffnung (164a, 286) an der distalen Wand (160, 280) erstreckt, um einen zweiten Schutzabschnitt (134) eines Sicherheitsschutzes (104, 242) zu ergreifen, der einen länglichen ersten Schutzabschnitt (136) aufweist, der so bemessen ist, dass er eine Nadel (108, 244) umschließt, die einen ersten Schaftabschnitt aufweist, der sich distal von der distalen Wand (160, 280) des Gehäuses (110, 248) erstreckt und einen zweiten Schaftabschnitt, der sich proximal von der distalen Wand (160, 280) des Gehäuses (110, 248) erstreckt;
eine Vorspannfeder (106, 246), die zwischen dem Sicherheitsschutz (104, 242) und dem Gehäuse (110, 248) zusammengedrückt ist; und
wobei der Aktuator (112, 250) zur distalen Wand (160, 280) des Gehäuses (110, 248) hin beweglich ist, um den zweiten Schutzabschnitt (134) vom Armabschnitt (176) zu lösen, um der Vorspannfeder (106, 246) zu ermöglichen, sich auszudehnen.

2. Blutentnahmenadelanordnung nach Anspruch 1, wobei das Gehäuse (110) einen Gehäuseaktuator (200) aufweist, der eine sich verjüngende Wand (202) aufweist, die sich proximal an der distalen Wand (160) erstreckt, um mit dem Arm (172a) des Aktuators (112) zu interagieren.

3. Blutentnahmenadelanordnung nach Anspruch 1 oder 2, wobei der Aktuator (112, 250) einen zylindrischen Körperabschnitt (167) aufweist, der eine distale Wand (168) mit einer Öffnung (170, 300) und einem offenen proximalen Ende (196) aufweist.

4. Blutentnahmenadelanordnung nach Anspruch 3, wobei der Arm (172a, 284) ein erster Arm (172a, 284) ist und sie ferner einen zweiten Arm (172b, 284) aufweist und wobei sich der erste Arm (172a, 284) und der zweite Arm (172b, 284) distal von der distalen Wand (168) des Aktuators (112, 250) erstrecken.

5. Blutentnahmenadelanordnung nach einem der Ansprüche 1 bis 4, ferner aufweisend eine Kappe (102), die konzentrisch um den Nadelschutz (104) und die Nadel (108) angeordnet ist.

6. Blutentnahmenadelanordnung nach Anspruch 5, wobei die Kappe (102) mindestens einen Federarm (132) aufweist, der eine erhabene Spitze (133) zum Anstoßen des zweiten Schutzabschnitts (134) in der Schutzstellung des Schutzschildes aufweist.

7. Blutentnahmenadelanordnung nach einem der Ansprüche 5 oder 6, wobei sich ein Teil des länglichen ersten Schutzabschnitts (136) distal an der Kappe (102) in einer Bereitstellungsposition erstreckt.

8. Blutentnahmenadelanordnung nach einem der Ansprüche 1 bis 7, ferner aufweisend einen Luer-Adapter zur mehrfachen Probennahme (114), der um den zweiten Schaftabschnitt angeordnet ist.

9. Blutentnahmenadelanordnung nach Anspruch 4, wobei sich der erste Arm (172a) und der zweite Arm (172b) jeweils durch eine Öffnung an der distalen Wand (160) des Gehäuses (110) erstrecken, um einen Flansch (140) auf dem zweiten Schutzabschnitt (134) zu ergreifen.

10. Blutentnahmenadelanordnung nach einem der Ansprüche 1 bis 9, wobei die Feder (106) zwischen einer Schulter (198) im zweiten Schutzabschnitt (134) und der distalen Wand (160) in einer Bereitstellungsposition zusammengedrückt ist.

11. Blutentnahmenadelanordnung (100, 240), aufweisend:
ein Gehäuse (102, 254), aufweisend einen Körper (156), der einen inneren Hohlraum aufweist und ein offenes proximales Ende zur Aufnahme eines, ein Septum aufweisenden, Vacutainers definiert;
ein Gehäuseaktuator (110, 248), aufweisend einen Körper (156, 282) und einen Unterbau (160, 280), der innerhalb des inneren Hohlraums des Gehäuses (102, 254) angeordnet ist, wobei der Unterbau (160, 280) einen Umfang, der mit einer inneren Oberfläche des inneren Hohlraumes in Stoßkontakt steht, einen Nadelhalter (162) zum Halten einer Nadel (108, 244), die ein erstes Schaftende und ein zweites Schaftende aufweist, und eine Öffnung (164a, 286), die von der Nadelsäule (162) absteht, aufweist;
einen Aktuator (112, 250), aufweisend eine Unterbaustange (167, 292) und mindestens einen Arm (172a, 284), der innerhalb des inneren Hohlraums des Gehäuses (102, 254) angeordnet ist und mindestens einen Arm (172a, 284) aufweist, der sich durch die Öffnung (164a, 286) auf dem Unterbau (160, 280) des Gehäuseaktuators (110, 248) erstreckt und wobei die Unterbaustange (167, 292) vom Unterbau (160, 280) des Gehäuseaktuators (110, 248) in einer Bereitstellungsposition beabstandet ist;
einen Nadelschutz (104, 242), der relativ zum Körper (156, 282) des Gehäuseaktuators (110, 248) verschiebbar angeordnet ist; und
eine Vorspannfeder (106, 246), die zwischen dem Nadelschutz (104, 242) und dem Gehäuseaktuator (110, 248) zusammengedrückt ist; und
wobei die Unterbaustange (167, 292) des Aktuators (112, 250) zum Unterbau (160, 280) des Gehäuseaktuators (110, 248) hin beweglich ist, um den Nadelschutz (104, 242) zu lösen, damit sich die Vorspannfeder (106, 246) ausdehnen kann.

12. Blutentnahmenadelanordnung nach Anspruch 11, wobei der Aktuator (112, 250) einen zweiten Arm (172b, 284) aufweist, der sich distal an der Unterbaustange (167, 292) erstreckt.

13. Blutentnahmenadelanordnung nach Anspruch 12, wobei sich der zweite Arm (172b, 284) durch eine zweite Öffnung (164b, 286) am Unterbau (160, 280) des Gehäuseaktuators (110, 248) erstreckt.

14. Blutentnahmenadelanordnung nach einem der Ansprüche 11 bis 13, wobei der Körper (156, 282) des Gehäuseaktuators (110, 248) im Allgemeinen zylindrisch ist und wobei der Nadelschutz (104, 242) zumindest teilweise im Inneren des Gehäuseaktuators (110, 248) angeordnet ist.

15. Blutentnahmenadelanordnung nach einem der Ansprüche 11 bis 14, wobei der Nadelschutz (242) mindestens zwei Blattfedern (268) mit jeweils einem Hakenende (266) aufweist und wobei jedes Hakenende (266) in eine Öffnung (272) eingreift, die am Körper (282) des Gehäuseaktuators (248) ausgebildet ist.

16. Blutentnahmenadelanordnung nach einem der Ansprüche 11 bis 15, wobei die Vorspannfeder (106, 246) zwei Enden aufweist und wobei ein erstes Ende der Feder (106, 246) durch eine Endwand des Nadelschutzes (104, 242) vorgespannt ist und ein zweites Ende der Feder (246) durch den Unterbau (160, 280) des Gehäuseaktuators (110, 248) vorgespannt ist.

17. Blutentnahmenadelanordnung nach einem der Ansprüche 12 bis 16, wobei der Arm (172a, 284) und der zweite Arm (172b, 284) des Aktuators (112, 250) jeweils einen Fortsatz (178, 288) aufweisen, der gegen den Körper (156, 282) des Gehäuseaktuators (110, 248) drückt.

18. Blutentnahmenadelanordnung nach einem der Ansprüche 11 bis 17, ferner aufweisend einen Luer-Adapter zur mehrfachen Probennahme (114, 252), der über dem zweiten Schaftende angeordnet ist.

19. Blutentnahmenadelanordnung nach Anspruch 18, wobei der Luer-Adapter zur mehrfachen Probennahme (114, 252) durch eine Öffnung (170, 300) an der Unterbaustange (167, 292) des Aktuators (112, 250) ragt.

20. Verfahren zum Herstellen einer Blutentnahmenadelanordnung (100, 240), wobei das Verfahren aufweist:
Bereitstellen eines Gehäuses (102, 254), aufweisend einen Körper (156), der einen inneren Hohlraum und ein offenes proximales Ende definiert, und einen Gehäuseaktuator (110, 248), aufweisend einen Körper (156, 282) und einen Unterbau (160, 280), der innerhalb des inneren Hohlraums des Gehäuses (102, 254) angeordnet ist;
Anlegen eines Umfangs des Unterbaus (160, 280) mit einer Innenfläche des inneren Hohlraums, wobei der Unterbau (160, 280) einen Nadelhalter (162) zum Halten einer Nadel (108, 244) aufweist, die ein erstes Schaftende und ein zweites Schaftende, und eine Öffnung (164a, 286), die von dem Nadelhalter (162) absteht, aufweist;
Anordnen eines Aktuators (112, 250), aufweisend mindestens einen Arm (172a, 284) innerhalb des inneren Hohlraums des Gehäuses (102, 254);
Erstrecken des mindestens einen Armes (172a, 284) durch die Öffnung (164a, 286) auf dem Unterbau (160, 280) des Gehäuseaktuators (110, 248);
Verschiebbares Anordnen eines Nadelschutzes (104, 242) relativ zum Körper (156, 282) des Gehäuseaktuators (110, 248); und
Zusammendrücken einer Vorspannfeder (106, 246) zwischen dem Nadelschutz (104, 242) und dem Gehäuseaktuator (110, 248); und
wobei der Aktuator (112, 250) zum Unterbau (160, 280) des Gehäuseaktuators (110, 248) hin beweglich ist, um den Nadelschutz (104, 242) zu lösen, damit sich die Vorspannfeder (106, 246) ausdehnen kann.

21. Verfahren nach Anspruch 20, wobei der Nadelschutz (242) mindestens zwei Blattfedern (268) mit jeweils einem Hakenende (266) aufweist und wobei jedes Hakenende (266) in eine Öffnung (272) eingreift, die am Körper (282) des Gehäuseaktuators (248) ausgebildet ist.

22. Verfahren nach einem der Ansprüche 20 bis 21, wobei der mindestens eine Arm (172a) einen zweiten Schutzabschnitt (134) des Nadelschutzes (104) greift, der einen länglichen ersten Schutzabschnitt (136) aufweist, der so bemessen ist, dass er eine Nadel (108) umgibt, die einen ersten Schaftabschnitt, der sich distal von der distalen Wand (160) des Gehäuses (110) erstreckt, und einen zweiten Schaftabschnitt, der sich proximal von der distalen Wand (160) des Gehäuses (110) erstreckt, aufweist.

## Revendications

1. Ensemble aiguille de prélèvement sanguin (100, 240) comprenant :
un boîtier (110, 248) comprenant un corps (156, 282) définissant une cavité intérieure et ayant une paroi distale (160, 280) avec une ouverture (164a, 286) et une extrémité proximale ouverte pour recevoir un vacutainer comprenant un septum ;
un actionneur (112, 250) situé dans la cavité intérieure du boîtier (110) et ayant un bras (172a, 284) avec une section de bras (176) s'étendant à travers l'ouverture (164a, 286) sur la paroi distale (160, 280) pour saisir une seconde section de protection (134) d'une protection de sécurité (104, 242), qui a une première section de protection allongée (136) dimensionnée pour entourer une aiguille (108, 244) ayant une première section d'arbre s'étendant de façon distale par rapport à la paroi distale (160, 280) du boîtier (110, 248) et une seconde section d'arbre s'étendant de façon proximale par rapport à la paroi distale (160, 280) du boîtier (110, 248) ;
un ressort de sollicitation (106, 246) comprimé entre la protection de sécurité (104, 242) et le boîtier (110, 248) ; et
l'actionneur (112, 250) étant mobile vers la paroi distale (160, 280) du boîtier (110, 248) pour libérer la seconde section de protection (134) de la section de bras (176) pour permettre au ressort de sollicitation (106, 246) de s'étendre.

2. Ensemble aiguille de prélèvement sanguin selon la revendication 1, dans lequel le boîtier (110) a un actionneur de boîtier (200) comprenant une paroi conique (202) s'étendant de façon proximale par rapport à la paroi distale (160) pour interagir avec le bras (172a) de l'actionneur (112).

3. Ensemble aiguille de prélèvement sanguin selon les revendications 1 ou 2, dans lequel l'actionneur (112, 250) comprend une section de corps cylindrique (167) comprenant une paroi distale (168) avec une ouverture (170, 300) et une extrémité proximale ouverte (196).

4. Ensemble aiguille de prélèvement sanguin selon la revendication 3, dans lequel le bras (172a, 284) est un premier bras (172a, 284) et comprenant en outre un second bras (172b, 284) et le premier bras (172a, 284) et le second bras (172b, 284) s'étendant de façon distale à partir de la paroi distale (168) de l'actionneur (112, 250).

5. Ensemble aiguille de prélèvement sanguin selon l'une quelconque des revendications 1 à 4, comprenant en outre un capuchon (102), disposé de façon concentrique autour de la protection d'aiguille (104) et de l'aiguille (108).

6. Ensemble aiguille de prélèvement sanguin selon la revendication 5, dans lequel le capuchon (102) comprend au moins un bras à ressort (132) ayant une pointe en saillie (133) pour venir en butée contre la seconde section de protection (134) dans la position de protection de la protection de sécurité.

7. Ensemble aiguille de prélèvement sanguin selon l'une quelconque des revendications 5 ou 6, dans lequel une partie de la première section de protection allongée (136) s'étend de façon distale par rapport au capuchon (102) dans une position prête.

8. Ensemble aiguille de prélèvement sanguin selon l'une quelconque des revendications 1 à 7, comprenant en outre un adaptateur Luer de type échantillonnage multiple (114) disposé autour de la seconde section d'arbre.

9. Ensemble aiguille de prélèvement sanguin selon la revendication 4, dans lequel le premier bras (172a) et le second bras (172b) s'étendent chacun à travers une ouverture au niveau de la paroi distale (160) du boîtier (110) pour saisir une bride (140) sur la seconde section de protection (134).

10. Ensemble aiguille de prélèvement sanguin selon l'une quelconque des revendications 1 à 9, dans lequel le ressort (106) est comprimé entre un épaulement (198) dans la seconde section de protection (134) et la paroi distale (160) dans une position prête.

11. Ensemble aiguille de prélèvement sanguin (100, 240) comprenant :
un boîtier (102, 254) comprenant un corps (156) définissant une cavité intérieure et une extrémité distale ouverte pour recevoir un vacutainer comprenant un septum ;
un actionneur de boîtier (110, 248) comprenant un corps (156, 282) et une base (160, 280) disposé dans la cavité intérieure du boîtier (102, 254), ladite base (160, 280) comprenant une circonférence en contact de butée avec une surface intérieure de la cavité intérieure, une tige d'aiguille (162) pour maintenir une aiguille (108, 244) comprenant une première extrémité d'arbre et une seconde extrémité d'arbre, et une ouverture (164a, 286) espacée de la tige d'aiguille (162) ;
un actionneur (112, 250) comprenant une barre de base (167, 292) et au moins un bras (172a, 284) situé dans la cavité intérieure du boîtier (102, 254) et ayant l'au moins un bras (172a, 284) s'étendant à travers l'ouverture (164a, 286) sur la base (160, 280) de l'actionneur de boîtier (110, 248) et la barre de base (167, 292) espacée de la base (160, 280) de l'actionneur de boîtier (110, 248) dans une position prête ;
une protection d'aiguille (104, 242) disposée de façon coulissante par rapport au corps (156, 282) de l'actionneur de boîtier (110, 248) ; et
un ressort de sollicitation (106, 246) comprimé entre la protection d'aiguille (104, 242) et l'actionneur de boîtier (110, 248) ; et
la barre de base (167, 292) de l'actionneur (112, 250) étant mobile vers la base (160, 280) de l'actionneur de boîtier (110, 248) pour libérer la protection d'aiguille (104, 242) pour permettre au ressort de sollicitation (106, 246) de s'étendre.

12. Ensemble aiguille de prélèvement sanguin selon la revendication 11, dans lequel l'actionneur (112, 250) comprend un second bras (172b, 284) s'étendant de façon distale par rapport à la barre de base (167, 292).

13. Ensemble aiguille de prélèvement sanguin selon la revendication 12, dans lequel le second bras (172b, 284) s'étend à travers une seconde ouverture (164b, 286) sur la base (160, 280) de l'actionneur de boîtier (110, 248).

14. Ensemble aiguille de prélèvement sanguin selon l'une quelconque des revendications 11 à 13, dans lequel le corps (156, 282) de l'actionneur de boîtier (110, 248) est généralement cylindrique et la protection d'aiguille (104, 242) étant disposée, au moins en partie, à l'intérieur de l'actionneur de boîtier (110, 248).

15. Ensemble aiguille de prélèvement sanguin selon l'une quelconque des revendications 11 à 14, dans lequel la protection d'aiguille (242) comprend au moins deux ressorts à lames (268) présentant chacun une extrémité de crochet (266) et chaque extrémité de crochet (266) étant en prise avec une ouverture (272) formée sur le corps (282) de l'actionneur de boîtier (248).

16. Ensemble aiguille de prélèvement sanguin selon l'une quelconque des revendications 11 à 15, dans lequel le ressort de sollicitation (106, 246) a deux extrémités et une première extrémité du ressort (106, 246) étant sollicitée par une paroi d'extrémité de la protection d'aiguille (104, 242) et une seconde extrémité du ressort (246) étant sollicitée par la base (160, 280) de l'actionneur de boîtier (110, 248).

17. Ensemble aiguille de prélèvement sanguin selon l'une quelconque des revendications 12 à 16, dans lequel le bras (172a, 284) et le second bras (172b, 284) de l'actionneur (112, 250) comprennent chacun une projection (178, 288) qui appuie sur le corps (156, 282) de l'actionneur de boîtier (110, 248).

18. Ensemble aiguille de prélèvement sanguin selon l'une quelconque des revendications 11 à 17, comprenant en outre un adaptateur Luer de type échantillonnage multiple (114, 252) positionné sur la seconde extrémité d'arbre.

19. Ensemble aiguille de prélèvement sanguin selon la revendication 18, dans lequel l'adaptateur Luer de type échantillonnage multiple (114, 252) fait saillie à travers une ouverture (170, 300) sur la barre de base (167, 292) de l'actionneur (112, 250).

20. Procédé de fabrication d'un ensemble aiguille de prélèvement sanguin (100, 240), le procédé comprenant :
la formation d'un boîtier (102, 254) comprenant un corps (156) définissant une cavité intérieure et une extrémité proximale ouverte, et un actionneur de boîtier (110, 248) comprenant un corps (156, 282) et une base (160, 280) disposé dans la cavité intérieure du boîtier (102, 254) ;
la mise en butée d'une circonférence de ladite base (160, 280) avec une surface intérieure de la cavité intérieure, la base (160, 280) comprenant une tige d'aiguille (162) pour maintenir une aiguille (108, 244) comprenant une première extrémité d'arbre et une seconde extrémité d'arbre, et une ouverture (164a, 286) espacée de la tige d'aiguille (162) ;
la localisation d'un actionneur (112, 250) comprenant au moins un bras (172a, 284) dans la cavité intérieure du boîtier (102, 254) ;
l'extension de l'au moins un bras (172a, 284) à travers l'ouverture (164a, 286) sur la base (160, 280) de l'actionneur de boîtier (110, 248) ;
la disposition coulissante d'une protection d'aiguille (104, 242) par rapport au corps (156, 282) de l'actionneur de boîtier (110, 248) ; et
la compression d'un ressort de sollicitation (106, 246) entre la protection d'aiguille (104, 242) et l'actionneur de boîtier (110, 248) ; et
l'actionneur (112, 250) étant mobile vers la base (160, 280) de l'actionneur de boîtier (110, 248) pour libérer la protection d'aiguille (104, 242) pour permettre au ressort de sollicitation (106, 246) de s'étendre.

21. Procédé selon la revendication 20, dans lequel la protection d'aiguille (242) comprend au moins deux ressorts à lames (268) présentant chacun une extrémité de crochet (266) et chaque extrémité de crochet (266) étant en prise avec une ouverture (272) formée sur le corps (282) de l'actionneur de boîtier (248).

22. Procédé selon l'une quelconque des revendications 20 à 21, dans lequel l'au moins un bras (172a) saisit une seconde section de protection (134) de la protection d'aiguille (104), qui a une première section de protection allongée (136) dimensionnée pour entourer une aiguille (108) ayant une première section d'arbre s'étendant de façon distale par rapport à la paroi distale (160) du boîtier (110) et une seconde section d'arbre s'étendant de façon proximale par rapport à la paroi distale (160) du boîtier (110).
